# EUROPÄISCHE PATENTSCHRIFT

(11) **EP 2 445 401 B1**
(45) Veröffentlichungstag und Bekanntmachung des Hinweises auf die Patenterteilung: **22.04.2015**
(21) Anmeldenummer: 10726952.4
(22) Anmeldetag: 22.06.2010
(51) Int. Cl.: A61B 5/06

(54) **BILDGEBUNGSVORRICHTUNG UND BILDGEBUNGSVERFAHREN ZUR AUFFINDUNG VON UNREGELMÄSSIGKEITEN IN HOHLRÄUMEN VON OBJEKTEN**
IMAGING DEVICE AND IMAGING METHOD FOR LOCATING IRREGULARITIES IN HOLLOW SPACES OF OBJECTS
DISPOSITIF D'IMAGERIE ET PROCÉDÉ D'IMAGERIE DESTINÉS À DÉTECTER DES IRRÉGULARITÉS DANS DES VOLUMES CREUX D'OBJETS

(30) Priorität: 24.06.2009 DE 102009030171
(43) Veröffentlichungstag der Anmeldung: 02.05.2012
(73) Patentinhaber: Fraunhofer-Gesellschaft zur Förderung der angewandten Forschung e.V., 80686 München (DE); Friedrich-Alexander-Universität Erlangen-Nürnberg, 91054 Erlangen (DE)
(72) Erfinder: MÜNZENMAYER, Christian, 91469 Hagenbüchach (DE); WITTENBERG, Thomas, 91054 Erlangen (DE); WINTER, Christian, 31141 Hildesheim (DE); SHEVCHENKO, Nikita, 80339 München (DE); BERGEN, Tobias, 91052 Erlangen (DE); RAITHEL, Martin, 90542 Eckenthal / Forth (DE)
(74) Vertreter: Rau, Schneck & Hübner Patentanwälte Rechtsanwälte PartGmbB
(86) Internationale Anmeldenummer: PCT/EP2010/058758
(87) Internationale Veröffentlichungsnummer: WO 2010/149635

(56) Entgegenhaltungen:
- WO-A2-2004/091361
- WO-A2-2006/027781
- US-A1- 2004 111 011
- US-A1- 2007 078 343

## Beschreibung

Die Erfindung betrifft eine Bildgebungsvorrichtung und ein entsprechendes Bildgebungsverfahren zur Auffindung von Unregelmäßigkeiten in Hohlräumen von Objekten.

Bildgebungsvorrichtungen, wie beispielsweise Videoskope, werden zur zerstörungsfreien Untersuchung von Objekten mit schwer zugänglichen Hohlräumen eingesetzt. Typische Anwendungsgebiete derartiger Bildgebungsvorrichtungen sind in der Technik die Inspektion von Bauteilhohlräumen, Maschinen sowie Rohrleitungen und in der Medizin die Untersuchung von Hohlorganen, wie beispielsweise dem Darm, der Atem- oder Harnwege. Nachteilig bei bekannten Bildgebungsvorrichtungen ist, dass einmal aufgefundene Unregelmäßigkeiten in den komplex geformten Hohlräumen nur schwer wiedergefunden werden können, so dass nachfolgende Untersuchungen der Unregelmäßigkeiten nur mit erheblichem Suchaufwand möglich sind.

Aus der WO 2004/091 361 A2 ist eine Bildgebungsvorrichtung mit einer Kapsel bekannt, in die ein bildgebender Sensor integriert ist. Die Position der Kapsel innerhalb eines Hohlraums eines Patienten wird mittels eines Tracking-Systems detektiert. Auf diese Weise können mittels einer ersten Kapsel diagnostische Informationen ermittelt und relevante Positionen markiert werden. Die markierten Positionen können anschließend wiederaufgefunden werden, um mit einer zweiten Kapsel eine Behandlung oder eine weitere Diagnose durchzuführen.

Aus der US 2004/111011 A1 ist eine Bildgebungsvorrichtung mit einer Kapsel bekannt, in die ein optischer Sensor integriert ist. Die Kapsel durchwandert den Darm eines Patienten und beginnt bei Erreichen einer ersten Position mit der Bildaufnahme, die bei Erreichen einer zweiten Position wieder beendet wird. Hierzu übermittelt die Kapsel Positionsdaten an eine Steuereinheit, die diese mit Daten einer gespeicherten Startposition und einer gespeicherten Stoppposition vergleicht.

Aus der WO 2006/027 781 A2 ist ein System zur Führung eines mit einem Katheter gekoppelten medizinischen Geräts an eine vordefinierte Position innerhalb eines Körperhohlraums eines Patienten bekannt. Hierzu werden bei der Führung des Katheters ermittelte Positionsdaten in Bezug auf die vordefinierte Position ausgewertet.

Aus der US 2007/078 343, A1 ist ein Ultraschallgerät mit einer führbaren Ultraschallsonde und einem Tracking-System bekannt. Mittels des Ultraschallgeräts sind bildgebende Daten und zugehörige Positionsdaten beim Auffinden von Unregelmäßigkeiten speicherbar.

Der Erfindung liegt daher die Aufgabe zugrunde, eine Bildgebungsvorrichtung zur Auffindung von Unregelmäßigkeiten in Hohlräumen von Objekten zu schaffen, die eine einfache und genaue Wiederauffindung der Unregelmäßigkeiten ermöglicht.

Diese Aufgabe wird durch eine Bildgebungsvorrichtung mit den Merkmalen des Anspruchs 1 gelöst. Erfindungsgemäß wurde erkannt, dass ein Wiederauffinden von Unregelmäßigkeiten in einfacher Weise möglich ist, wenn Positionsdaten des bildgebenden Sensors mittels einer Positionsmesseinrichtung in einem Bezugskoordinatensystem ständig ermittelt werden und bei jedem erstmaligen Auffinden einer Unregelmäßigkeit anhand der aufgenommenen bildgebenden Daten mittels der Steuereinheit eine Speicherung von Ziel-Positionsdaten ausgelöst wird. Die Ziel-Positionsdaten beschreiben die Position des bildgebenden Sensors in dem Bezugskoordinatensystem zum Zeitpunkt der Aufnahme der bildgebenden Daten, die zur Auffindung der Unregelmäßigkeit geführt haben. Die gespeicherten Ziel-Positionsdaten geben für die Wiederauffindung der gesuchten Unregelmäßigkeit mittels des führbar ausgebildeten bildgebenden Sensors die anzufahrenden Koordinaten in dem Bezugskoordinatensystem an. Die Vergleichseinheit führt bei der Wiederauffindung der Unregelmäßigkeit in periodischen Zeitabständen einen Vergleich zwischen den gespeicherten Ziel-Positionsdaten und den aktuell ermittelten Positionsdaten durch und stellt dem Benutzer geeignete Informationen zur Wiederauffindung der Unregelmäßigkeit bereit. Werden die Ziel-Positionsdaten objektspezifisch in einer nicht-flüchtig ausgebildeten Speichereinheit gespeichert, so können Unregelmäßigkeiten einer Vielzahl von Objekten auch in großen Zeitintervallen wiedergefunden werden.

Das Zusammenwirken der Registriereinheit mit der Vergleichseinheit ermöglicht das Erkennen und Ausgleichen von Objektbewegungen beim Wiederauffinden von Unregelmäßigkeiten. Beispielsweise wird der Bewegungspfad, der bei einer ersten Untersuchung von dem bildgebenden Sensor zurückgelegt und mittels der Auswerteeinrichtung aufgezeichnet wurde, mit dem Bewegungspfad bei einer nachfolgenden Untersuchung abgeglichen, wodurch die veränderte Position des Objekts in dem Bezugskoordinatensystem bei der nachfolgenden Untersuchung ermittelt wird und die Ziel-Positionsdaten entsprechend korrigiert werden können. In der Registriereinheit ist hierzu ein Registrierverfahren implementiert, das eine Koordinatentransformation zwischen den Bewegungspfaden errechnet. Derartige Registrierverfahren sind aus der digitalen Bildverarbeitung grundsätzlich bekannt. Die endseitige Anordnung des führbaren bildgebenden Sensors an einer flexiblen Führung ermöglicht eine Führung des bildgebenden Sensors in komplexen tubulären Hohlräumen.

Eine Bildgebungsvorrichtung nach Anspruch 2 ermöglicht eine vollautomatische Auffindung und Wiederauffindung von Unregelmäßigkeiten. In der Detektionseinheit ist ein geeignetes Detektionsverfahren implementiert, das die digitalen bildgebenden Daten analysiert und Unregelmäßigkeiten, wie beispielsweise Materialfehler oder Läsionen, erkennt. Derartige Detektionsverfahren sind aus der digitalen Bildverarbeitung prinzipiell bekannt. Mittels Texturverfahren und/oder Segmentierungsverfahren können Oberflächen- und/oder Formmerkmale der Unregelmäßigkeiten in den bildgebenden Daten analysiert und detektiert werden.

Eine Bildgebungsvorrichtung nach Anspruch 3 ermöglicht ein einfaches Auffinden von Unregelmäßigkeiten durch einen Benutzer. Der Benutzer kann die Detektionseinheit nach Anspruch 2 entweder ersetzen oder ergänzen. Die bildgebenden Daten werden auf dem Monitor in Echtzeit visualisiert, so dass der Benutzer auf dem Monitor nach Unregelmäßigkeiten suchen kann. Erkennt dieser eine Unregelmäßigkeit auf dem Monitor, so betätigt er das Eingabegerät und es wird durch die Steuereinheit ein Speichervorgang ausgelöst. Darüber hinaus kann der Benutzer mittels der Detektionseinheit automatisch detektierte Unregelmäßigkeiten auf dem Monitor einer Kontrolle unterziehen und Fehldetektionen mittels des Eingabegerätes korrigieren.

Eine Bildgebungsvorrichtung nach Anspruch 4 stellt sicher, dass zu Ziel-Positionsdaten auch die zugehörigen bildgebenden Daten, die zur Speicherung der Ziel-Positionsdaten geführt haben, gespeichert und mit den Ziel-Positionsdaten verknüpft werden. Hierdurch kann während einer Untersuchung oder bei nachfolgenden Untersuchungen die wiederaufgefundene Unregelmäßigkeit mit den gespeicherten bildgebenden Daten verglichen werden, wodurch Veränderungen festgestellt werden können. Dies ist insbesondere bei medizinischen Untersuchungen von großer Bedeutung. Im Falle einer Darmspiegelung können so Polypen, die beim Vorspiegeln entdeckt wurden, beim Rückzug wieder aufgefunden und mit den gespeicherten bildgebenden Daten verglichen werden.

Eine Bildgebungsvorrichtung nach Anspruch 5 ermöglicht ein schnelleres Wiederauffinden von Unregelmäßigkeiten. Der Benutzer kann anhand des visualisierten Objektmodells den bildgebenden Sensor gezielt zu den gekennzeichneten Ziel-Orten in dem Objektmodell führen. Das visualisierte Objektmödell erlaubt somit eine direktere Führung des bildgebenden Sensors zu dem gewünschten Ziel-Ort und somit ein intuitives Einstellen der Ziel-Positionsdaten.

Eine Bildgehungsvorrichtung nach Anspruch 6 erhöht die Genauigkeit beim Wiederauffinden von Unregelmäßigkeiten. Weisen die zu untersuchenden Objekte prinzipiell die gleiche Form, jedoch eine unterschiedliche Größe und/oder unterschiedliche Deformationen auf, so kann in der Speichereinheit ein Standard-Objektmodell gespeichert werden, das anhand der ermittelten Positionsdaten - einmalig oder mehrmalig - skaliert und/oder auf die ermittelten Positionsdaten registriert wird. Das Standard-Objektmodell wird so auf die tatsächliche Größe und/oder Form des Objekts angepasst. Die in dem Objektmodell gekennzeichneten Ziel-Orte entsprechen aufgrund der Skalierung mit einer größeren Genauigkeit dem tatsächlichen Ort der Unregelmäßigkeit. Dies ist insbesondere bei der Untersuchung von Hohlorganen von Bedeutung, da ein individuelles Modell des zu untersuchenden Hohlorgans nur mit hohem Aufwand ermittelbar ist, jedoch Standardmodelle vorliegen.

Eine Bildgebungsvorrichtung nach Anspruch 7 vereinfacht das Wiederauffinden von Unregelmäßigkeiten. Sobald die aktuell ermittelten Positionsdaten vordefinierte Abstandsschwellwerte zu den Ziel-Positionsdaten unterschreiten und dementsprechend weitestgehend mit den Ziel-Positionsdaten übereinstimmen, wird ein Signal für das Wiederauffinden der Unregelmäßigkeit erzeugt. Das Signal kann beispielsweise eine optische Einblendung auf dem Monitor oder einen akustischen Signalton generieren.

Eine Bildgebungsvorrichtung nach Anspruch 8 ermöglicht eine Visualisierung von Bewegungspfaden des bildgebenden Sensors Das Absuchen der Hohlräume wird hierdurch vereinfacht.

Eine Bildgebungsvorrichtung nach Anspruch 9 ermöglicht einen Vergleich des Bewegungspfades mit dem Objektmodell. Durch diesen Vergleich können insbesondere Bewegungen des bildgebenden Sensors, die nicht auf eine entsprechende Formung des Objekts zurückzuführen sind, erkannt werden. Somit können ungewollte Schlaufenbildungen von Führungen, an denen der bildgebenden Sensor angeordnet ist, erkannt und rückgängig gemacht werden. Darüber hinaus erlauben die Bewegungspfade eine Vorhersage der Bewegungsrichtung und dementsprechend eine Aussage darüber, ob ein gewünschter Ziel-Ort erreicht wird.

Eine Bildgebungsvorrichtung nach Anspruch 10 eignet sich für Vorsorgeuntersuchungen des Magen-Darm-Traktes. Die zu untersuchende Person nimmt zunächst die Kapsel ein. Während diese den Magen-Darm-Trakt durchwandert, nimmt der erste bildgebende Sensor in periodischen Zeitabständen bildgebende Daten auf und übermittelt diese an die Auswerteeinrichtung. Die übermittelten bildgebenden Daten können in der bereits beschriebenen Weise ausgewertet und Unregelmäßigkeiten detektiert werden. Nachdem die Kapsel ausgeschieden wurde, können die aufgefundenen Unregelmäßigkeiten mittels des zweiten bildgebenden Sensors genauer untersucht werden. Hierzu wird dieser mittels der flexiblen Führung an den Ziel-Positionsdaten positioniert, so dass die entsprechende Unregelmäßigkeit einfach wieder aufgefunden und weiter untersucht werden kann.

Eine Bildgebungsvorrichtung nach Anspruch 11 ermöglicht eine einfache und genaue Positionsermittlung. Eine Bildgebungsvorrichtung nach Anspruch 12 gewährleistet eine sichere Detektion von Unregelmäßigkeiten. Je nach Bedarf kann der Kamera-Sensor für Strahlung im Ultraviolett-Bereich, im sichtbaren Bereich oder im Infrarot-Bereich ausgelegt sein.

Der Erfindung liegt ferner die Aufgabe zugrunde, ein Bildgebungsverfahren zur Auffindung von Unregelmäßigkeiten in Hohlräumen von technischen Objekten zu schaffen, das eine einfache und genaue Wiederauffindung der Unregelmäßigkeiten ermöglicht.

Diese Aufgabe wird erfindungsgemäß durch ein Bildgebungsverfahren mit den Merkmalen des Anspruches 13 gelöst. Die Vorteile des erfindungsgemäßen Verfahrens entsprechen den bereits beschriebenen Vorteilen der erfindungsgemäßen Vorrichtung.

Weitere Merkmale, Vorteile und Einzelheiten der Erfindung ergeben sich aus der nachfolgenden Beschreibung mehrerer Ausführungsbeispiele anhand der Zeichnung. Es zeigen:
- Fig. 1: eine Prinzipdarstellung einer Bildgebungsvorrichtung gemäß einem ersten Ausführungsbeispiel,
- Fig. 2: eine Prinzipdarstellung der Bildgebungsvorrichtung in Fig. 1 bei der Wiederauffindung von Unregelmäßigkeiten eines zu untersuchenden Objektes,
- Fig. 3: eine Prinzipdarstellung von zwei mit der Bildgebungsvorrichtung in Fig. 2 zurückgelegten Bewegungspfaden bei der Auffindung und Wiederauffindung von Unregelmäßigkeiten, und
- Fig. 4: eine Prinzipdarstellung einer Bildgebungsvorrichtung gemäß einem zweiten Ausführungsbeispiel.

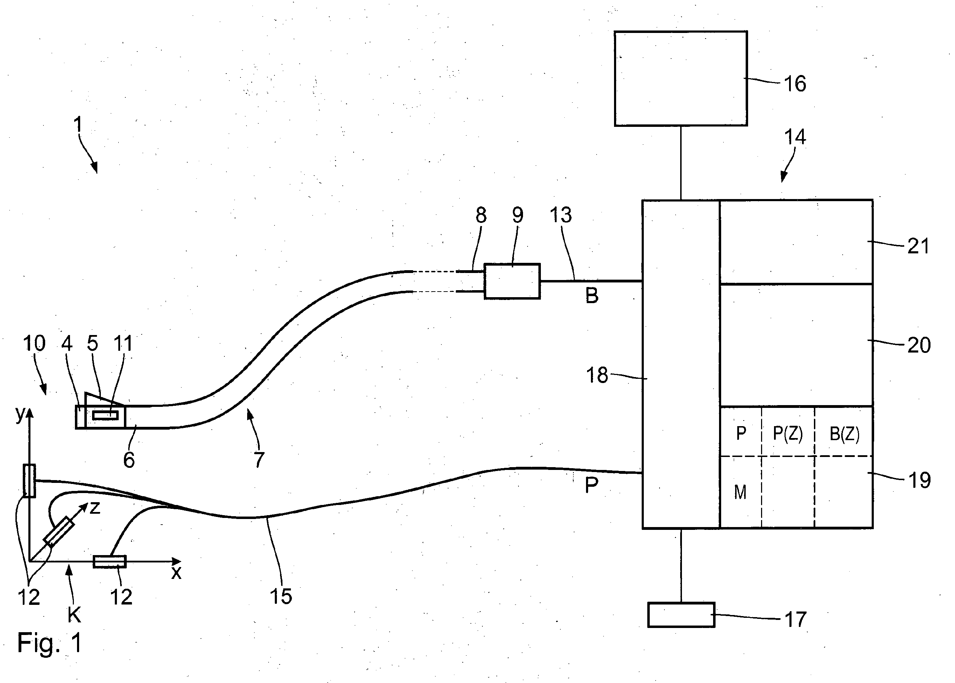

Nachfolgend wird anhand der Fig. 1 bis 3 ein erstes Ausführungsbeispiel der Erfindung beschrieben. Die in Fig. 1 gezeigte Bildgebungsvorrichtung 1 dient zur Untersuchung von Objekten 2 und zum Auffinden von Unregelmäßigkeiten Z in von diesen Objekten 2 gebildeten Hohlräumen 3. Die Bildgebungsvorrichtung 1 ist nach Art eines Koloskops ausgebildet und wird für die Durchführung von Vorsorgeuntersuchungen des Dickdarms eingesetzt. Der Dickdarm stellt für diesen Anwendungsfall das zu untersuchende Objekt 2 dar.

Die Bildgebungsvorrichtung 1 weist einen bildgebenden Sensor 4 auf, der zusammen mit einem Strahlungsaustritt 5 an einem ersten Ende 6 einer flexiblen Führung 7 angeordnet ist. An einem zweiten Ende 8 der Führung 7 ist ein Griff 9 befestigt, an dem mehrere nicht näher-dargestellte Bedienelemente angeordnet sind. Der Strahlungsaustritt 5 ist beispielsweise über eine in der Führung angeordnete Glasfaserleitung mit einer nicht dargestellten-Strahlungsquelle verbunden. Die Führung 7 ist schlauchförmig ausgebildet und beinhaltet beispielsweise Energieversorgungs- und Datenübertragungsleitungen für den Sensor 4 und eine Glasfaserleitung zur Übertragung von Strahlung zu dem Strahlungsaustritt 5.

Zur Ermittlung der Position des bildgebenden Sensors 4 in einem Bezugskoordinatensystem K weist die Bildgebungsvorrichtung 1 eine Positionsmesseinrichtung 10 auf. Die Positionsmesseinrichtung 10 ist elektromagnetisch ausgebildet und umfasst einen als Magnetspule ausgebildeten Sender 11 und mehrere als Antennen ausgebildete Empfänger 12. Der Sender 11 ist im Bereich des ersten Endes 6 - nahe dem Sensor 4 - in der Führung 7 angeordnet. Die zugehörigen Leitungen verlaufen ebenfalls in der Führung 7. Die Empfänger 12 sind räumlich beabstandet zueinander um den Sender 11 angeordnet, so dass mittels diesen die Position des Senders 11 in dem Bezugskoordinatensystem K ermittelbar ist.

Der bildgebende Sensor 4 ist zur Übertragung der aufgenommenen bildgebenden Daten B über eine erste Datenübertragungsleitung 13 mit einer Auswerteeinrichtung 14 verbunden. Entsprechend sind die Empfänger 12 zur Übertragung der gewonnenen Positionsdaten P für die Position des Senders 11 über eine zweite Datenübertragungsleitung 15 mit der Auswerteeinrichtung 14 verbunden.

Die Auswerteeinrichtung 14 dient zur Auswertung der bildgebenden Daten B und ist beispielsweise als PC ausgebildet. Die Auswerteeinrichtung 14 umfasst einen Monitor 16, ein Eingabegerät 17, eine Steuereinheit 18, eine Speichereinheit 19, eine Registriereinheit 20 und eine Vergleichseinheit 21.

Die Speichereinheit 19 ist beispielsweise nicht-flüchtig ausgebildet und dient zur digitalen Speicherung von Daten, wie beispielsweise der bildgebenden Daten B oder der Positionsdaten P, so dass die gespeicherten Daten sowohl während einer laufenden Untersuchung als auch bei einer Wiederholungsuntersuchung zur Verfügung stehen. Die Speichereinheit 19 wird mittels der Steuereinheit 18 gesteuert, die bei der Auffindung einer Unregelmäßigkeit Z anhand der bildgebenden Daten B(Z) die Speicherung von Ziel-Positionsdaten P(Z) auslöst. Das Auslösen eines Speichervorgangs kann beispielsweise automatisch durch eine Detektionseinheit erfolgen, in der ein geeignetes bildbasiertes Detektionsverfahren implementiert ist, und/oder durch das von einem Benutzer betätigte Eingabegerät 17. Das Eingabegerät 17 kann beispielsweise als Fußschalter, Handschalter oder Handtaster ausgebildet sein. Die Ziel-Positionsdaten P(Z) beschreiben die Position des Senders 11 und aufgrund der räumlichen Nähe des Sensors 4 zum Zeitpunkt der Aufnahme der bildgebenden Daten B(Z), anhand der die Unregelmäßigkeit Z aufgefunden wurde.

In der Speichereinheit 19 sind Modelldaten eines Objektmodells M gespeichert. Die Modelldaten beschreiben ein Standard-Dickdarm-Modell, das den Dickdarm einer Standard-Person wiedergibt.

Die Funktionsweise der Bildgebungsvorrichtung 1 ist wie folgt:
Der bildgebende Sensor 4 wird an der den Schließmuskel kennzeichnenden Stelle A in den Dickdarm 2 eingeführt und mittels der Führung 7 in Richtung des Dünndarms D bewegt. Bei dieser Bewegung beschreibt der Sensor 4 einen ersten Bewegungspfad T₁, der als Hin-Pfad bezeichnet wird. Der Sensor 4 nimmt in periodischen Zeitabständen Δt bildgebende Daten B auf, die an die Auswerteeinrichtung 14 übertragen und mittels der Steuereinheit 18 auf dem Monitor 16 visualisiert werden. Die Positionsmesseinrichtung 10 ermittelt zu entsprechenden Zeitpunkten Positionsdaten P des Sensors 4 und überträgt diese an die Auswerteeinrichtung 14. Die Positionsdaten P werden in der Speichereinheit 19 gespeichert.

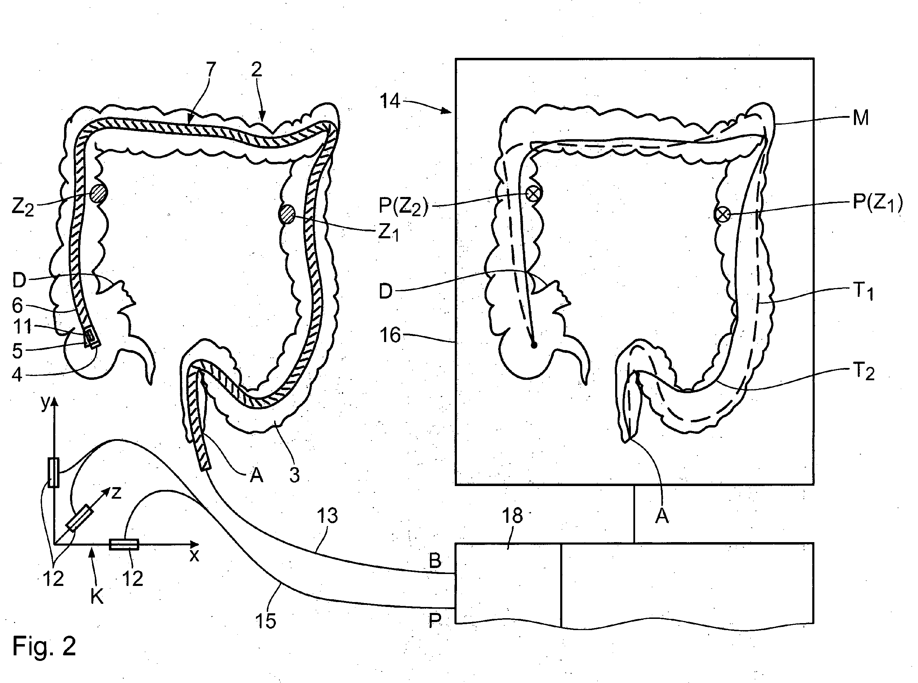

Erkennt der Benutzer der Bildgebungsvorrichtung 1 auf dem Monitor 16 eine Unregelmäßigkeit Z, so betätigt er das Eingabegerät 17. Das Eingabegerät 17 wirkt mit der Steuereinheit 18 zusammen, die einen Speichervorgang der Speichereinheit 19 auslöst, so dass die aktuell visualisierten bildgebenden Daten B(Z), die die Unregelmäßigkeit Z zeigen, sowie die zugehörigen Positionsdaten P(Z) einander zugeordiiet in der Speichereinheit 19 gespeichert werden. Fig. 2 zeigt zwei Unregelmäßigkeiten Z₁ und Z₂, die auf dem Hin-Pfad T₁ aufgefunden und entsprechende Positionsdaten P(Z₁) und P(Z₂) gespeichert wurden. Typische Unregelmäßigkeiten Z bei der Untersuchung des Dickdarms 2 sind Polypen oder andere Läsionen.

Die eigentliche Untersuchung des Dickdarms 2 erfolgt bei der Bewegung des Sensors 4 von den Dünndarm D zu dem Schließmuskel A zurück. Der dabei von dem Sensor 4 beschriebene zweite Bewegungspfad T₂ wird als Rück-Pfad bezeichnet.

Die auf dem Rück-Pfad T₂ aufgenommenen bildgebenden Daten B werden in entsprechender Weise an die Auswerteeinrichtung 14 übertragen und visualisiert. In entsprechender Weise werden die Positionsdaten P des Sensors 4 an die Auswerteeinrichtung 14 übermittelt und in der Speichereinheit 19 gespeichert. Erkennt der Benutzer eine Unregelmäßigkeit Z, so können die zugehörigen bildgebenden Daten B(Z) sowie die Ziel-Positionsdaten P(Z) durch Betätigen des Eingabegeräts 17 in der Speichereinheit 19 gespeichert werden.

Die auf dem Hin-Pfad T₁ aufgefundenen Unregelmäßigkeiten Z₁ und Z₂ können auf dem Rück-Pfad T₂ aufgrund einer Faltenbildung des Dickdarms 2 verdeckt und so für eine genauere Untersuchung schwer auffindbar sein. Zur Wiederauffindung der Unregelmäßigkeiten Z₁ und Z₂ vergleicht die Vergleichseinheit 21 die aktuell ermittelten Positionsdaten P mit den abgespeicherten Ziel-Positionsdaten P(Z₁) und P(Z₂) und erzeugt bei weitestgehender Übereinstimmung, wenn also vordefinierte Abstandsschwellwerte unterschritten werden, ein Signal für die Wiederauffindung der jeweiligen Unregelmäßigkeit Z₁ bzw. Z₂. Die Unregelmäßigkeiten Z₁ und Z₂ können somit auch dann auf dem Rück-Pfad T₂ wiederaufgefunden werden, wenn diese durch eine Faltenbildung des Dickdarms 2 verdeckt sind. Das Signal kann eine optische und/oder akustische Warnung generieren. Die optische Warnung kann beispielsweise auf dem Monitor 16 angezeigt werden.

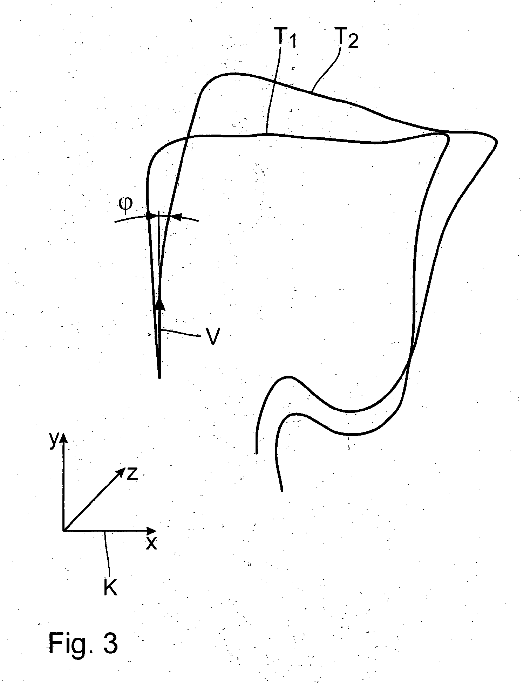

Durch Positionsveränderungen des Dickdarms 2 relativ zu dem Bezugskoordinatensystem K werden die Ziel-Positionsdaten P(Z) für das Wiederauffinden der Unregelmäßigkeiten Z verfälscht. Um derartige Positionsveränderungen berücksichtigen zu können, werden die Positionsdaten P₁ des Hin-Pfades T₁ sowie die Positionsdaten P₂ des Rück-Pfades T₂ der Registriereinheit 20 zugeführt. Die Registriereinheit 20 führt mittels eines implementierten Registrierverfahrens einen Abgleich des Rück-Pfades T₂ mit dem Hin-Pfad T₁ durch. Durch diesen Abgleich werden Positionsveränderungen, wie beispielsweise translatorische Verschiebungen und/oder Rotationen, ermittelt und eine Koordinatentransformation zwischen den Positionsdaten des Hin-Pfades T₁ und des Rück-Pfades T₂ berechnet. Die Registriereinheit 20 wirkt derart mit der Vergleichseinheit 21 zusammen, dass anhand der berechneten Koordinatentransformation der Vergleich der aktuell ermittelten Positionsdaten P mit den Ziel-Positionsdaten P(Z) korrigiert wird und so die Unregelmäßigkeiten Z mit einer höheren Genauigkeit wieder aufgefunden werden können. Fig. 3 zeigt beispielhaft zwei Bewegungspfade T₁ und T₂, die aufgrund einer Positionsveränderung des Dickdarms 2 um den Vektor V zueinander translatorisch verschoben und um den Winkel ϕ in der x-y-Ebene zueinander gedreht sind. Mittels des Vektors V und des Winkels ϕ kann eine Koordinatentransformation in dem Bezugskoordinatensystem K durchgeführt werden.

Der Stand der Untersuchung kann jederzeit bei Bedarf, beispielsweise durch entsprechendes Betätigen des Eingabegerätes 17, auf dem Monitor 16 visualisiert werden. Hierzu lädt die Steuereinheit 18 aus der Speichereinheit 19 die Positionsdaten P₁ des Hin-Pfades T₁ sowie die Positionsdaten P₂ des Rück-Pfades T₂, die Ziel-Positionsdaten P(Z₁) und P(Z₂) sowie die Modelldaten des Objektmodells M und überträgt diese an den Monitor 16, der eine entsprechende Visualisierung durchführt. Da es sich bei dem Objektmodell M um ein Standard-Objektmodell handelt, wird dieses bei der Visualisierung anhand der ermittelten Positionsdaten P skaliert. Das Objektmodell M ist - je nach Stand der Untersuchung - somit mit den Bewegungspfaden T₁ und T₂ sowie mit den den Ziel-Positionsdaten P(Z) entsprechenden Ziel-Orten aller Unregelmäßigkeiten Z versehen. Die Ziel-Orte können beispielsweise durch entsprechende Markierungen im Objektmodell M visualisiert werden. An den Ziel-Orten kann bei Bedarf, beispielsweise durch entsprechendes Betätigen des Eingabegeräts 17, ein aus den bildgebenden Daten B(Z) generiertes Bild der zugehörigen Unregelmäßigkeit Z eingeblendet werden.

Alternativ kann der Monitor 16 eine zweigeteilte Visualisierung von Daten derart ermöglichen, dass in einem ersten Teil des Monitors 16 die aktuell aufgenommenen bildgebenden Daten B des Sensors 4 visualisiert werden und in einem zweiten Teil das den Stand der Untersuchung zeigende Objektmodell M mit eingeblendeten Markierungen für die Unregelmäßigkeiten Z₁ und Z₂ visualisiert wird.

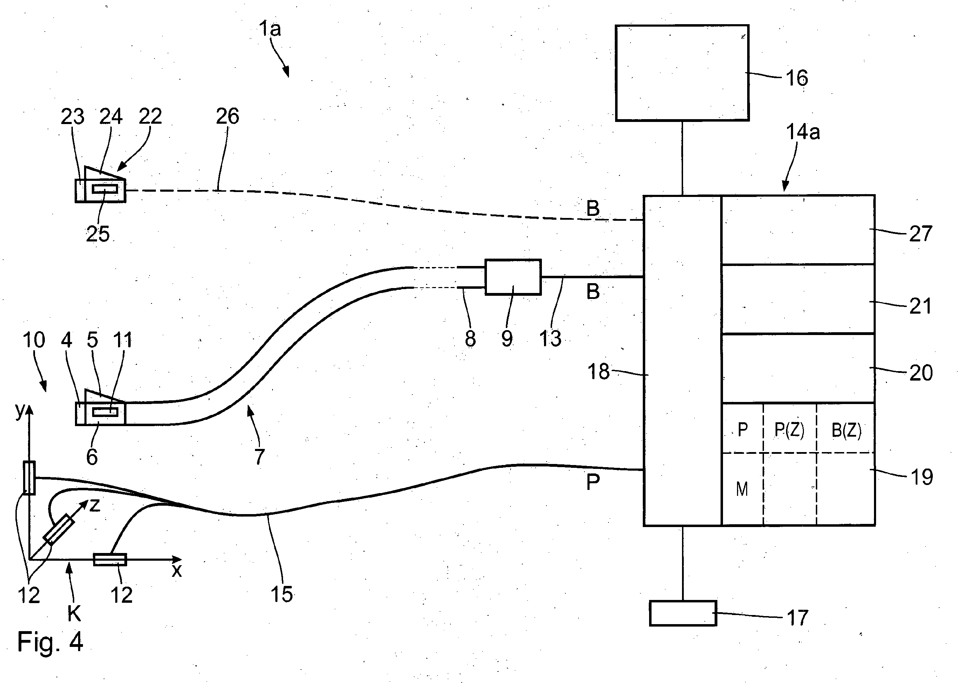

Nachfolgend wird unter Bezugnahme auf Fig. 4 ein zweites Ausführungsbeispiel der Erfindung beschrieben. Konstruktiv identische Teile erhalten dieselben Bezugszeichen wie bei dem ersten Ausführungsbeispiel, auf dessen Beschreibung hiermit verwiesen wird. Konstruktiv unterschiedliche, jedoch funktionell gleichartige Teile erhalten dieselben Bezugszeichen mit einem nachgestellten a. Die Bildgebungsvorrichtung 1a weist gegenüber dem ersten Ausführungsbeispiel zusätzlich eine Kapsel 22 auf, die mit einem zweiten bildgebenden Sensor 23, einer zugehörigen Strahlungsquelle 24 und einem zweiten Sender 25 für die Positionsmesseinrichtung 10 versehen ist. Die Energieversorgung der Kapsel 22 erfolgt mittels einer nicht dargestellten Batterie. Die Übertragung der aufgenommenen bildgebenden Daten B zu der Auswerteeinrichtung 14a erfolgt drahtlos, was in Fig. 4 den gestrichelt eingezeichneten Datenübertragungskanal 26 angedeutet ist.

Die Auswerteeinrichtung 14a weist zusätzlich eine Detektionseinheit 27 auf, in die ein Detektionsverfahren implementiert ist, das anhand von Textur- und/oder Formmerkmalen aus den bildgebenden Daten B Unregelmäßigkeiten Z automatisch detektiert.

Die zu untersuchende Person nimmt zunächst die Kapsel 22 ein. Ausgehend von dem Dünndarm D durchwandert die Kapsel 22 den Dickdarm 2 in Richtung des Schließmuskels A. Der in der Kapsel 22 integrierte bildgebende Sensor 23 nimmt in periodischen Zeitabständen Δt bildgebende Daten B auf und übermittelt diese drahtlos über den Datenübertragungskanal 26 an die Auswerteeinrichtung 14a. Die bildgebenden Daten B werden der Detektionseinheit 27 zugeführt, die mittels des implementierten Detektionsverfahrens automatisch nach Unregelmäßigkeiten Z sucht.

In einer vollautomatischen Ausführungsform der Auswerteeinrichtung 14a wirkt die Detektionseinheit 27 derart mit der Steuereinheit 18 zusammen, dass bei einer automatischen Auffindung einer Unregelmäßigkeit Z unmittelbar ein Speichervorgang ausgelöst wird, so dass die bildgebenden Daten B(Z) sowie die zugehörigen Ziel-Positionsdaten P(Z) in der Speichereinheit 19 abgespeichert werden.

In einer halbautomatischen Ausführungsform der Auswerteeinrichtung 14a wirkt die Detektionseinheit 27 derart mit der Steuereinheit 18 zusammen, dass bei der automatischen Auffindung einer Unregelmäßigkeit Z diese zunächst mittels der zugehörigen bildgebenden Daten B(Z) auf dem Monitor 16 visualisiert wird. Der Benutzer muss zur Auslösung eines Speichervorgangs die aufgefundene Unregelmäßigkeit Z als solche bestätigen. Hierzu muss das Eingabegerät 17 entsprechend betätigt werden. Durch wie Betätigung des Eingabegeräts 17 werden die bildgebenden Daten B(Z) und die zugehörigen Ziel-Positionsdaten P(Z) in der Speichereinheit 19 gespeichert.

Nach dem Ausscheiden der Kapsel 22 erfolgt die eigentliche Untersuchung der Unregelmäßigkeiten Z. Hierzu wird der an der Führung 7 angeordnete bildgebende Sensor 4 über den Schließmuskel A in den Dickdarm 2 eingeführt. Die Untersuchung erfolgt entsprechend dem ersten Ausführungsbeispiel, wobei aufgrund der mittels der Kapsel 22 bereits gewonnenen Informationen die Untersuchung gezielter und schneller erfolgen kann. Hinsichtlich der weiteren Funktionsweise wird auf das erste Ausführungsbeispiel verwiesen.

Die Bildgebungsvorrichtungen 1, 1a speichern die Ziel-Positionsdaten P(Z) vorzugsweise objekt- und/oder personenspezifisch, so dass einmal aufgefundene Unregelmäßigkeiten Z auch bei nachfolgenden Untersuchungen in größeren Zeitintervallen wiedergefunden werden können.

Die Positionsmesseinrichtung 10 kann beispielsweise als mechanisches, elektromagnetisches oder bildbasiertes Trackingsystem ausgebildet sein. Ein mechanisches Trackingsystem kann beispielsweise durch Krümmungssensoren in der flexiblen Führung 7 realisiert werden, so dass der Verlauf der Führung 7 und die Position des Sensors 4 berechnet werden kann. Hinsichtlich des elektromagnetischen Trackingsystems wird auf die beschriebenen Ausführungsbeispiele verwiesen.

Der bildgebende Sensor 4, 23 kann - je nach Bedarf- als zweidimensionaler Kamera-Sensor bzw. -Chip für Strahlung im Ultraviolett-Bereich, Infrarot-Bereich oder Sichtbereich oder als Ultraschallsensor ausgebildet sein. Die Strahlungsquelle mit dem Strahlungsaustritt 5 ist zur Ausleuchtung des zu untersuchenden Hohlraums 3 entsprechend an den Sensor 4, 23 angepasst.

Das Eingabegerät 17 kann zur mechanischen und/oder akustischen Eingabe von Informationen ausgebildet sein. Zur mechanischen Eingabe kann das Eingabegerät 17 beispielsweise als Fußschalter, Handschalter oder Handtaster ausgebildet sein. Zur akustischen Eingabe kann das Eingabegerät 17 beispielsweise als Mikrofon mit entsprechender Sprach- bzw. Kommando-Erkennung ausgebildet sein. Darüber hinaus kann das Eingabegerät 17 auch zur optischen Eingabe von Informationen ausgebildet sein und beispielsweise eine Kamera mit einer Gestik-Erkennung umfassen.

Als Objektmodell M kann beispielsweise bei Hohlorganen ein vereinfachtes generisches Anatomiemodell verwendet werden. Weiterhin kann ein personenspezifisches Objektmodell M aus präoperativen CT-Daten oder aus endoskopischen Bilddäten generiert werden. Bei Bauteilen kann ein Objektmodell M beispielsweise aus CAD-Daten gewonnen werden. Das Objektmodell M kann zusammen mit allen relevanten Informationen, wie beispielsweise den Ziel-Positionsdaten P(Z), den Bewegungspfaden T und weiteren anatomisch bzw. technisch relevanten Positionen, visualisiert werden.

Die Bildgebungsvorrichtung 1, 1a kann beispielsweise als Videoskop bzw. Endoskop oder Koloskop ausgebildet sein und in der Industrie zur Inspektion von technischen Hohlräumen 3, wie beispielsweise Bauteilhohlräumen oder Rohrleitungen, sowie in der Medizin zur Lokalisierung von Läsionen in Hohlorganen bzw. tubulären Hohlräumen, wie beispielsweise dem Dickdarm, dem Dünndarm, der Speiseröhre, der Blase oder der Lunge eingesetzt werden.

Die Bildgebungsvorrichtungen 1, 1a gemäß den beschriebenen Ausführungsbeispielen können auch zur Inspektion von technischen Objekten 2 eingesetzt werden. Insbesondere kann die Kapsel 22 auch technische Hohlräume 3 autonom durchwandern. Die Detektionseinheit 27 in der voll- und halbautomatischen Ausführungsform kann auch in dem ersten Ausführungsbeispiel Verwendung finden.

Durch die Erfindung wird das Wiederauffinden von Unregelmäßigkeiten Z erleichtert. Die Untersuchungsergebnisse können besser dokumentiert und standardisiert werden. So können beispielsweise bei jeder Untersuchung einer bestimmten Unregelmäßigkeit Z bildgebende Daten B(Z) gespeichert und miteinander bzw. mit Referenzbildem verglichen werden.

## Patentansprüche

1. Bildgebungsvorrichtung zur Auffindung von Unregelmäßigkeiten in Hohlräumen von Objekten mit
- mindestens einem bildgebenden Sensor (4; 4, 23) zur Aufnahme von bildgebenden Daten (B),
- einer Positionsmesseinrichtung (10) zur Ermittlung von Positionsdaten (P) des mindestens einen bildgebenden Sensors (4; 4, 23), und
- einer Auswerteeinrichtung (14; 14a) zur Auswertung der bildgebenden Daten (B), umfassend
-- eine Speichereinheit (19) zur digitalen Speicherung von Daten (B, P),
-- eine Steuereinheit (18) zur Auslösung einer Speicherung von Ziel-Positionsdaten (P(Z)) bei der Auffindung einer Unregelmäßigkeit (Z) anhand der bildgebenden Daten (B), und
-- einer Vergleichseinheit (21) zum Vergleich der gespeicherten Ziel-Positionsdaten (P(Z)) mit aktuell ermittelten Positionsdaten (P) bei der Wiederauffindung der Unregelmäßigkeit (Z) mittels des mindestens einen bildgebenden Sensors (4; 4)
**dadurch gekennzeichnet, dass**
- zumindest ein bildgebender Sensor (4; 4) zur gezielten Positionierung in einem Hohlraum (3) endseitig an einer flexiblen Führung (7) angeordnet und führbar ist, und
- die Auswerteeinrichtung (14; 14a) eine Registriereinheit (20) umfasst, die derart mit der Vergleichseinheit (21) zusammenwirkt, dass anhand eines Abgleichs von Bewegungspfaden (T) der Vergleich der Ziel-Positionsdaten (P(Z)) mit den ermittelten Positionsdaten (P) korrigiert wird.

2. Bildgebungsvorrichtung nach Anspruch 1, **dadurch gekennzeichnet, dass** die Auswerteeinrichtung (14a) eine Detektionseinheit (27) umfasst, die derart mit der Steuereinheit (18) zusammenwirkt, dass bei einer automatischen Auffindung einer Unregelmäßigkeit (Z) anhand von bildgebenden Daten (B) zugehörige Ziel-Positionsdaten (P(Z)) in der Speichereinheit (19) gespeichert werden.

3. Bildgebungsvorrichtung nach Anspruch 1 oder 2, **dadurch gekennzeichnet, dass** die Auswerteeinrichtung (14; 14a) einen Monitor (16) und ein Eingabegerät (17) umfasst, die derart mit der Steuereinheit (18) zusammenwirken, dass die bildgebenden Daten (B) auf dem Monitor (16) visualisiert werden und durch Betätigung des Eingabegerätes (17) der Steuereinheit (18) die Auffindung einer Unregelmäßigkeit (Z) angezeigt wird und zugehörige Ziel-Positionsdaten (P(Z)) in der Speichereinheit (19) gespeichert werden.

4. Bildgebungsvorrichtung nach einem der Ansprüche 1 bis 3, **dadurch gekennzeichnet, dass** die Steuereinheit (18) derart ausgebildet ist, dass zu den Ziel-Positionsdaten (P(Z)) die zugehörigen bildgebenden Daten (B(Z)) in der Speichereinheit (19) gespeichert werden.

5. Bildgebungsvorrichtung nach einem der Ansprüche 1 bis 4, **dadurch gekennzeichnet, dass** die Steuereinheit (18) derart ausgebildet ist, dass ein in der Speichereinheit (19) gespeichertes Objektmodell (M) auf einem Monitor (16) visualisiert und die den Ziel-Positionsdaten (P(Z)) entsprechenden Ziel-Orte in dem Objektmodell (M) gekennzeichnet werden.

6. Bildgebungsvorrichtung nach Anspruch 5, **dadurch gekennzeichnet, dass** die Steuereinheit (18) derart ausgebildet ist, dass das gespeicherte Objektmodell (M) anhand von ermittelten Positionsdaten (P) angepasst wird.

7. Bildgebungsvorrichtung nach einem der Ansprüche 1 bis 6, **dadurch gekennzeichnet, dass** die Vergleichseinheit (21) derart ausgebildet ist, dass bei Übereinstimmung der Ziet-Positionsdaten(P(Z)) mit den ermittelten Positionsdaten (P) ein Signal für die Wiederauffindung der Unregelmäßigkeit (Z) erzeugt wird.

8. Bildgebungsvorrichtung nach einem der Ansprüche 1 bis 7, **dadurch gekennzeichnet, dass** die Steuereinheit (18) derart ausgebildet ist, dass die ermittelten Positionsdaten (P) zur Erstellung von Bewegungspfaden (T) in der Speichereinheit (19) gespeichert werden.

9. Bildgebungsvorrichtung nach Anspruch 8, **dadurch gekennzeichnet, dass** die Steuereinheit (18) derart ausgebildet ist, dass die gespeicherten Positionsdaten (P) in Form eines Bewegungspfades (T) in dem Objektmodell (M) gekennzeichnet werden.

10. Bildgebungsvorrichtung nach einem der Ansprüche 1 bis 9, **dadurch gekennzeichnet, dass** ein erster bildgebender Sensor (23) zur Auffindung einer Unregelmäßigkeit (Z) an einer Kapsel (22) und ein zweiter bildgebender Sensor (4) zur Wiederauffindung der Unregelmäßigkeit (Z) endseitig an einer flexiblen Führung (7) angeordnet ist.

11. Bildgebungsvorrichtung nach einem der Ansprüche 1 bis 10, **dadurch gekennzeichnet, dass** die Positionsmesseinrichtung (10) elektromagnetisch ausgebildet ist.

12. Bildgebungsvorrichtung nach einem der Ansprüche 1 bis 11, **dadurch gekennzeichnet, dass** der mindestens eine bildgebende Sensor (4; 4, 23) als Kamera-Sensor ausgebildet ist.

13. Bildgebungsverfahren zur Auffindung von Unregelmäßigkeiten in Hohlräumen von technischen Objekten, umfassend die Schritte:
- Aufnehmen von bildgebenden Daten (B) mittels mindestens eines sich in einem Hohlraum (3) befindlichen bildgebenden Sensors (4; 23),
- Ermitteln von Positionsdaten (P) des mindestens einen bildgebenden Sensors (4; 23) mittels einer Positionsmesseinrichtung (10),
- Auffinden einer Unregelmäßigkeit (Z) anhand der bildgebenden Daten (B),
- Auslösen einer Speicherung von Ziel-Positionsdaten (P(Z)) mittels einer Steuereinheit (18) beim Auffinden der Unregelmäßigkeit (Z),
- Speichern der Ziel-Positionsdaten (P(Z)) in einer Speichereinheit (19), und
- geführtes Positionieren des mindestens einen endseitig an einer flexiblen Führung (7) angeordneten und führbaren bildgebenden Sensors (4; 4) anhand eines mittels einer Vergleichseinheit (21) durchgeführten Vergleichs der gespeicherten Ziel-Positionsdaten (P(Z)) mit aktuell ermittelten Positionsdaten (P) zum Wiederauffinden der Unregelmäßigkeit (Z), wobei eine Registriereinheit (20) derart mit der Vergleichseinheit (21) zusammenwirkt, dass anhand eines Abgleichs von Bewegungspfaden (T) der Vergleich der Ziel-Positionsdaten (P(Z)) mit den ermittelten Positionsdaten (P) korrigiert wird.

## Claims

1. Imaging device for detecting irregularities in hollow spaces of objects, comprising
- at least one imaging sensor (4; 4, 23) for capturing imaging data (B),
- a position measuring device (10) for determining positioning data (P) of the at least one imaging sensor (4; 4, 23), and
- an evaluation device (14; 14a) for evaluating the imaging data (B), comprising
-- a storage unit (19) for the digital storing of data (B, P),
-- a control unit (18) for triggering a storing of target positioning data (P(Z)) when an irregularity (Z) is detected by means of the imaging data (B), and
-- a comparison unit (21) for comparing the stored target positioning data (P(Z)) with currently detected positioning data (P) when the irregularity (Z) is detected again by means of the at least one imaging sensor (4; 4),
**characterised in that**
- at least one imaging sensor (4; 4) is arranged at the end of a flexible guide to be guidable for targeted positioning in a hollow space (3), and
- the evaluation unit (14; 14a) comprises a recording device (20) that interacts with the comparison unit (21) in such a way that the comparison of the target positioning data (P(Z)) with the detected positioning data (P) is corrected by means of a comparison of trajectories (T).

2. Imaging device according to claim 1, **characterised in that** the evaluation unit (14a) comprises a detection unit (27) that interacts with the control unit (18) in such a way that when an irregularity (Z) is detected automatically by means of imaging data (B), associated target positioning data (P(Z)) are stored in the storage unit (19).

3. Imaging device according to claim 1 or 2, **characterised in that** the evaluation unit (14; 14a) comprises a screen (16) and an input device (17) that interact with the control unit (18) in such a way that the imaging data (B) are visualised on the screen (16), and the detection of an irregularity (Z) is displayed by actuating the input device (17) of the control unit (18), and associated target positioning data (P(Z)) are stored in the storage unit (19).

4. Imaging device according to one of claims 1 to 3, **characterised in that** the control unit (18) is configured in such a way that the imaging data (B(Z)) associated with the target positioning data (P(Z)) are stored in the storage unit (19).

5. Imaging device according to one of claims 1 to 4, **characterised in that** the storage unit (18) is configured in such a way that an object model (M) stored in the storage unit (19) is visualised on a screen (16), and the target positions corresponding to the target positioning data (P(Z) are marked in the object model (M).

6. Imaging device according to claim 5, **characterised in that** the control unit (18) is configured in such a way that the stored object model (M) is updated by means of determined positioning data (P).

7. Imaging device according to one of claims 1 to 6, **characterised in that** the comparison unit (21) is configured in such a way that when the target positioning data (P(Z)) are identical to the determined positioning data (P), a signal is generated which allows the irregularity (Z) to be detected again.

8. Imaging device according to one of claims 1 to 7, **characterised in that** the control unit (18) is configured in such a way that the determined positioning data (P) are stored in the storage unit (19) in order to generate trajectories (T).

9. Imaging device according to claim 8, **characterised in that** the control unit (18) is configured in such a way that the stored positioning data (P) are marked in the object model (M) in the form of a trajectory (T).

10. Imaging device according to one of claims 1 to 9, **characterised in that** a first imaging sensor (23) for detecting an irregularity (Z) is arranged on a capsule (22) while a second imaging sensor (4) for detecting the irregularity (Z) again is arranged at the end of a flexible guide (7).

11. Imaging device according to one of claims 1 to 10, **characterised in that** the position measuring device (10) is electromagnetic.

12. Imaging device according to one of claims 1 to 11, **characterised in that** the at least one imaging sensor (4; 4, 23) is an image sensor.

13. Imaging method for detecting irregularities in hollow spaces of technical objects, comprising the steps of
- capturing imaging data (B) by means of at least one imaging sensor (4; 23) disposed in a hollow space (3),
- determining positioning data (B) of the at least one imaging sensor (4; 23) by means of a position measuring device (10),
- detecting an irregularity (Z) by means of the imaging data (B),
- triggering a storing of target positioning data (P(Z)) by means of a control unit (18) when the irregularity (Z) is being detected,
- storing the target positioning data (P(Z)) in a storage unit (19), and
- positioning the at least one sensor (4; 4), which is guidably arranged at the end of a flexible guide (7), in a guided manner by means of a comparison, carried out by means of a comparison unit (21), of the stored target positioning data (P(Z)) with currently determined positioning data (P) to detect the irregularity (Z) again, wherein a recording device (20) interacts with the comparison unit (21) in such a way that the comparison of the target positioning data (P(Z)) with the determined positioning data (P) is corrected by means of a comparison of trajectories (T).

## Revendications

1. Dispositif d'imagerie servant à retrouver des irrégularités dans des volumes creux d'objets comprenant
- au moins un détecteur d'imagerie (4 ; 4, 23) pour l'enregistrement de données d'imagerie (B),
- un système de mesure de position (10) pour l'évaluation de données de position (P) d'au moins un détecteur d'imagerie (4 ; 4, 23), et
- un système d'exploitation (14 ; 14a) pour le traitement des données d'imagerie (B) étant constitué
-- d'une unité de mémoire (19) pour la mémorisation digitalisée de données (B, P),
-- d'une unité de commande (18) pour la libération d'une mémorisation de données de positions cibles (P(Z)) lors de la découverte d'une irrégularité (Z) à l'aide des données d'imagerie (B), et
-- d'une unité de comparaison (21) pour la comparaison des données de positions cibles (P(Z)) mémorisées avec des données de position (P) déterminées à l'instant lors de la découverte renouvelée de l'irrégularité (Z) au moyen d'au moins un détecteur d'imagerie (4 ; 4)
**caractérisé en ce**
- **qu'**au moins un détecteur d'imagerie est disposé et peut être déplacé au niveau de l'extrémité d'un guide (7) flexible pour un positionnement ciblé dans un volume creux (3), et
- **que** le système d'exploitation (14 ; 14a) comporte une unité d'enregistrement (20), qui agit conjointement avec l'unité de comparaison (21), de telle manière que la comparaison des positions de données cibles (P(Z)) avec les données de position (P) est corrigée à l'aide d'une comparaison de trajectoires (T).

2. Dispositif d'imagerie selon la revendication 1, **caractérisé en ce que** le système d'exploitation (14a) comporte une unité de détection (27), qui agit conjointement avec l'unité de commande (18) de telle manière que, lors d'une détection automatique d'une irrégularité (Z) à l'aide de données d'imagerie (B), les positions cibles (P(Z)) correspondantes sont mémorisées dans l'unité de mémoire (19).

3. Dispositif d'imagerie selon les revendications 1 ou 2, **caractérisé en ce que** le système d'exploitation (14 ; 14a) comporte un moniteur (16) et un appareil d'affichage (17), qui interagissent avec l'unité de commande (18) de telle manière que les données d'imagerie (B) sont visualisées sur le moniteur (16) et sont affichées, et la découverte d'une irrégularité (Z) est indiquée par la confirmation de l'appareil d'affichage (17) de l'unité de commande (18) et les données de position cible (P(Z)) correspondantes sont mémorisées dans l'unité de mémorisation (19).

4. Dispositif d'imagerie selon l'une des revendications 1 à 3, **caractérisé en ce que** l'unité de commande (18) est conçue de telle manière que les données d'imagerie (B(Z)) correspondant aux données de positions cibles (P(Z)) sont mémorisées dans l'unité de mémorisation (19).

5. Dispositif d'imagerie selon l'une des revendications 1 à 4, **caractérisé en ce que** l'unité de commande (18) est conçue de telle manière qu'un modèle de l'objet (M) mémorisé dans l'unité de mémorisation (19) est visualisé sur un moniteur (16) et les lieux cibles correspondant aux données de positions cibles (P(Z)) sont caractérisés dans le modèle de l'objet (M).

6. Dispositif d'imagerie selon la revendication 5, **caractérisé en ce que** l'unité de commande (18) est conçue de telle manière que le modèle de l'objet (M) mémorisé est mis au point à l'aide des données de position (P) déterminées.

7. Dispositif d'imagerie selon l'une des revendications 1 à 6, **caractérisé en ce que** l'unité de comparaison (21) est conçue de telle manière qu'un signal est émis pour une nouvelle découverte de l'irrégularité (Z) lorsque les données de position cible (P(Z)) sont identiques aux données de position (P) déterminées.

8. Dispositif d'imagerie selon l'une des revendications 1 à 7, **caractérisé en ce que** l'unité de commande (18) est conçue de telle manière que les données de position (P) déterminées sont mémorisées dans l'unité de mémorisation (19) pour l'établissement de trajectoires (T).

9. Dispositif d'imagerie selon la revendication 8, **caractérisé en ce que** l'unité de commande (18), est conçue de telle manière que les données de position mémorisées (P) sont caractérisées dans le modèle de l'objet (M) sous la forme d'une trajectoire (T).

10. Dispositif d'imagerie selon l'une des revendications 1 à 9, **caractérisé en ce qu'**un premier détecteur d'imagerie (23) est disposé pour la découverte d'une irrégularité (Z) sur une capsule (22) et qu'un deuxième détecteur d'imagerie (4) est disposé pour la découverte renouvelée de l'irrégularité (Z) à l'extrémité d'un guide (7) flexible.

11. Dispositif d'imagerie selon l'une des revendications 1 à 10, **caractérisé en ce que** le système de mesure de position (10) est conçu électromagnétique.

12. Dispositif d'imagerie selon l'une des revendications 1 à 11, **caractérisé en ce qu'**au moins le détecteur d'imagerie (4 ; 4, 23) est conçu sous la forme d'un détecteur photographique.

13. Procédé d'imagerie destiné à détecter des irrégularités dans des volumes creux d'objets techniques, comprenant les étapes :
- de saisie de données d'imagerie (B) au moyen d'au moins un détecteur d'imagerie (4 ; 4, 23) se situant dans un volume creux (3),
- de détermination de données de position (P) d'au moins un détecteur d'imagerie (4 ; 23) au moyen d'un système de mesure de position (10),
- de recherche d'une irrégularité (Z) à l'aide des données d'imagerie (B),
- de libération d'une mémoire de données de positions cibles (P(Z)) au moyen d'une unité de commande (18) lors de la découverte de l'irrégularité (Z),
- de mémorisation des données de positions cibles (P(Z)) dans une unité de mémorisation (19), et
- de positionnement guidé d'au moins un détecteur d'imagerie (4 ; 4) disposé et pouvant être guidé une extrémité d'un guide (7) flexible à l'aide d'une comparaison des données de position (P(Z)) mémorisées avec des données de positions déterminées à l'instant au moyen de l'unité de comparaison (21) pour une nouvelle découverte de l'irrégularité (Z), une unité d'enregistrement (20) agissant conjointement avec l'unité de comparaison (21) de sorte qu'avec l'aide d'une comparaison de trajectoires (T), la comparaison des données de positions cibles (P(Z)) est corrigée avec les données de position (P) déterminées.
